# EUROPEAN PATENT APPLICATION

(11) **EP 4 649 939 A1**
(43) Date of publication of application: **19.11.2025**
(21) Application number: 25175418.0
(22) Date of filing: 09.05.2025
(51) Int. Cl.: A61K 8/31, A61K 9/00, A61K 8/67, A61Q 5/00, A61K 8/34, A61K 31/045, A61K 47/06, A61K 47/44, A61Q 5/10

(54) **USE OF A COSMETIC COMPOSITION FOR MAINTAINING AND/OR RESTORING THE SCALP MICROBIOME**

(30) Priority: 17.05.2024 EP 24176521; 28.06.2024 EP 24185370
(71) Applicant: Wella Germany GmbH, 64295 Darmstadt (DE)
(72) Inventor: TAN, Chun Wei, 64295 Darmstadt (DE); KOH, Angel, 64295 Darmstadt (DE)
(74) Representative: Bandpay & Greuter

(57) **Abstract**

The presently claimed invention relates to the use of a cosmetic composition for maintain the scalp microbiome during and/or after chemical hair treatment or restoring the scalp microbiome after chemical hair treatment, and the related method for treating hair. The cosmetic composition comprises at least a mixture of at least two liquid oils being at least one liquid fatty acid oil and at least one liquid hydrocarbon oil, and at least one solid fat and/or wax.

## Description

### Field

The presently claimed invention relates to the use of a cosmetic composition after chemical hair treatment for maintaining the scalp microbiome during and/or after chemical hair treatment, or for restoring the scalp microbiome, and the related method for chemically treating hair. The cosmetic composition comprises at least a mixture of at least two liquid oils and at least one solid fat and/or wax.

### Background

A microbiome is a community of microorganisms, including bacteria, yeasts, viruses, and fungi, that exist in given environment. It is defined by the microbiome diversity including the taxonomy and the absolute/relative abundance. Microorganisms that are present on human skin are referred as skin flora or skin microbiome. Specifically, microorganisms that are present on human scalp are referred as scalp flora or scalp microbiome. These microorganisms are mostly found on the superficial layers of the epidermis and the upper parts of hair follicles.

The skin microbiome naturally comprises non-pathogenic microorganisms, which may be commensal microorganisms (not harmful to their host) or mutualistic microorganisms (providing a benefit to the host).

For example, it has been reported that prominent bacteria that exist on the scalp are bacteria of the genus *Staphylococcus, Propionibacterium* (also known as *Cutibacterium*)*, Corynebacterium, Bacillus* and *Pseudomonas,* and that prominent fungal species are *Malassezia, Aspergillus, Cladosporium, Wallemia* and *Alternaria* (see the article from Saxena et al., entitled "Comparison of healthy and dandruff scalp microbiome reveals the role of commensals in scalp health", Front Cell Infect Microbiology, 2018, 8:346; the article from Grimshaw et al., entitled "The diversity and abundance of fungi and bacteria on the healthy and dandruff affected human scalp", PLoS One, 2019, 14(12), e0225796).

Among these, non-pathogenic microorganisms colonizing the scalp are for example *Staphylococcus epidermidis* (associated with a beneficial role in acne), *Staphylococcus capitis* (associated with strong antibacterial activity against gram-negative bacteria, such as *Staphylococcus aureus*)*, Cutibacterium granulosum* (associated with the maintenance of the normal skin barrier and the stimulation of the host immune system), *Cutibacterium acnes* (associated with the maintenance of the acidic skin pH via breakdown of sebum lipids to free fatty acids, thereby inhibiting the growth of *Staphylococcus aureus -* see the article from E. A. Grice et al. entititled "The skin microbiome", Nature Reviews Microbiology, 2011, 9, 244-253) and *Malassezia globosa.*

The skin microbiome, particularly the scalp microbiome, may also comprise pathogenic or opportunistic pathogenic microorganisms *i.e.,* microorganisms colonizing the skin, which are known to be associated with particular health conditions.

For example, among these, pathogenic microorganisms colonizing the scalp are *Staphylococcus aureus* (associated with psoriasis and dermatitis), *Pseudomonas aeruginosa* (associated with psoriasis, folliculitis), *Malassezia restricta* (associated with dandruff) (see the article from Saxena et. al, entitled "Longitudinal study of the scalp microbiome suggests coconut oil to enrich healthy scalp commensals", Nature, open access, 31 March 2021)

The presence of non-pathogenic microorganisms and/or pathogenic microorganisms on skin, particularly on scalp, their quantity and relative proportions depend on different factors including the host (including the age, gender, genetics), his/her skin health (including the presence or absence of skin diseases, allergies, genetic predispositions/susceptibilities, the immune system strength, hormone and/or the use of medication), the environment (including the climate, UV exposure, and/or pollution) as well his/her physical activities, hygiene, beauty routine, stress, and exposure to chemicals. An imbalance, a disruption and/or an alteration of the microbiome is referred as dysbiosis. Dysbiosis may lead to conditions including acne, atopic dermatitis, dry skin, dandruff, some forms of eczema.

Particularly, hygiene and beauty routine may negatively impact the skin microbiome, particularly the scalp microbiome, for example by altering its diversity or absolute/relative abundance, changing the functional composition and the metabolic activities of the microorganisms, unbalancing it in favor of pathogenic microorganisms, lengthening its recovery, increasing the risk of scalp reactions (including inflammation, acne, atopic dermatitis, psoriasis, rosacea and/or allergies). Such impact depends on the products used, their frequency of use, the mode of application, etc.

Human hair may be treated in many different ways, including by hair shampooing, hair conditioning, hair grooming, hair dyeing, hair bleaching, hair straightening and/or hair perming. Some of these treatments, particularly chemical hair treatments, that may lead to a temporary, semi-permanent or a permanent alteration of hair, may be harsh thereby causing discomfort, irritation or even damages on hair and/or on skin. More generally, chemical hair treatments may negatively impact the scalp microbiome, for example because of a variation in pH and/or temperature.

For example, the permanent alteration of the color of human hair, by application of hair dyes is well known. In order to provide the consumer with the hair color and the intensity of color desired, a very complex chemical process is utilized. Permanent hair dyeing compositions usually comprise developers (also known as oxidative hair dye precursors or primary intermediates) and couplers (also known as color modifiers or secondary intermediates). Developers are sufficiently small to diffuse into the hair shaft where, once activated by an oxidizing agent, such as hydrogen peroxide, the developers react with other dye compounds *e.g*., the couplers to form larger colored complexes - chromophores - in the hair shaft. Couplers, which are also sufficiently small to diffuse into the hair shaft, are generally colorless molecules that form colors in the presence of activated developers. One or more developers can be used in combination with one or more couplers.

Because of the use of a reactive oxidative chemistry, usually at alkali pH, hair dyeing, particularly semi-permanent hair dyeing and permanent hair dyeing, often has a negative impact on the scalp microbiome. Hair dyeing may sensitize the scalp. During and/or after hair dyeing, the scalp microbiome may become imbalanced, possibly leading to dysbiosis. The scalp microbiome may also need several days before starting to recover.

In an attempt to limit the skin sensitization, particularly scalp sensitization, induced by chemical hair treatments, different skincare/haircare solutions are known. For example, it is known to apply a composition containing anti-inflammatory agents; and/or skin-soothing agents such as panthenol, dipotassium glycyrrhizate, allantoin (to soothe the inflammation caused by hair dyeing agents); and/or pain-receptor antagonists such as 4-t-butylcyclohexanol (to reduce stinging sensation). However, these skincare/haircare solutions are usually not sufficiently satisfactory, as they do not allow to maintain a balanced scalp microbiome (prevention) and/or to restore a balanced scalp microbiome (treatment). For example, a composition comprising anti-inflammatory agents and/or skin-soothing agents usually requires to be repeatedly applied over a long period of time to provide the expected skin benefit by penetration into the skin. However, such water-based composition, which is applied after dyeing hair, is usually not sufficient to protect the skin from dyeing agents. A composition comprising pain-receptor antagonists usually only has the potential to increase the pain tolerance of the user causing them to perceive a typically harmful sensation as innocuous.

In order to maintain or (re)balance the skin microbiome, other strategies are known, for example the use of prebiotic agents such as alpha-glucan oligosaccharides or inulin; the use of probiotic agents such as *bifidobacterium* or *lactobacillus;* and/or the use of postbiotic agents such as *lactobacillus* ferment or *bifida* ferment lysate. These agents may be used to modulate the skin microbiome when incorporated into a suitable cosmetic composition. However, such composition requires multiple applications over a long period of time.

There is therefore the need for providing a cosmetic composition, which maintains the skin microbiome during and/or after the chemical hair treatment or which restores the skin microbiome after the hair treatment, particularly which maintains the scalp microbiome during and/or after the hair dyeing or restores the scalp microbiome after the hair dyeing.

Accordingly, it is an object of the presently claimed invention to use a cosmetic composition for maintaining the scalp microbiome during and/or after chemical hair treatment or restoring the scalp microbiome after chemical hair treatment; wherein the cosmetic composition comprises at least two liquid oils being at least one liquid fatty acid oil and at least one liquid hydrocarbon oil; wherein the at least two liquid oils are present in a total amount in the range of ≥ 40 to ≤ 95 wt.-%, related to the total weight of the composition; wherein the at least one liquid hydrocarbon oil and at least one fatty acid oil are present in a weight ratio in the range of ≥ 1:1 to ≤ 140:1; wherein the composition comprises at least one solid fat and/or wax chosen from the group consisting of non-volatile hydrocarbons, solid hydrocarbon esters or mixtures thereof; and wherein the at least one solid fat and/or wax is present in an amount in the range of ≥ 5 to ≤ 60 wt.-%, related to the total weight of the composition.

Accordingly, it is also an object of the presently claimed invention to provide a method for chemically treating hair and skin, particularly hair and scalp, comprising the following steps: applying a cosmetic composition (e.g., on the skin and/or on hair roots); chemically treating hair; and, rinsing the chemically treated hair.

### Summary

The inventors surprisingly found that the application of the claimed cosmetic composition helps protecting the scalp, particularly the scalp microbiome, against the detrimental effect of chemical hair treatments. Overall, the application of the claimed cosmetic composition helps protecting the skin, particularly the skin microbiome, against the detrimental effect of chemical hair treatments. Particularly, the application of the composition before chemical hair treatment, particularly before hair dyeing, helps preventing or at least limiting dysbiosis *i.e.,* the appearance of an imbalanced scalp microbiome during and/or after the chemical hair treatment, particularly the hair dyeing. The application of the composition before chemical hair treatment, particularly before hair dyeing, thus helps maintaining the scalp microbiome *(i.e.,* a balanced scalp microbiome referred as eubiosis) during and/or after the chemical hair treatment or restoring the scalp microbiome (*i.e.,* a balanced scalp microbiome referred as eubiosis) after the chemical hair treatment. The same applies to alternative skin portions, particularly hairy skin portions, for example men's bearded cheeks and hairy chests. The same also applies to alternative chemical hair treatments including hair bleaching, hair straightening and hair perming. The composition is thus suitable for use as a skincare (scalp-care) and haircare composition. Without wishing to be bound by any theory, it is believed that the application of the cosmetic composition may help in protecting the skin microbiome and the skin condition as a whole, keeping it in a healthier state when subject to treatments such as a treatment with hair dyeing compositions. This results in increasing or at least maintaining the absolute and/or relative abundance, particularly the absolute abundance, of non-pathogenic microorganisms (such as *Cutibacterium acnes, Cutibacterium granulosum, Staphylococcus capitis, Malassezia globosa*) and the microbial diversity on the skin after chemical hair treatment application, thereby preventing or at least limiting the growth of pathogenic microorganisms, preventing or at least limiting the reduction of the biological functions that the skin microbiome contributes to skin homeostasis, and/or may increase the availability of the nutrients for the non-pathogenic microorganisms. However, it does not act as a prebiotic. The recovery rate of the non-pathogenic microorganisms may therefore increase.

Hence, in an aspect, the presently claimed invention relates to the use of a cosmetic composition for maintaining the scalp microbiome during and/or after chemical hair treatment or restoring the scalp microbiome after chemical hair treatment; wherein the cosmetic composition comprises at least two liquid oils being at least one liquid fatty acid oil and at least one liquid hydrocarbon oil; wherein the at least two liquid oils are present in a total amount in the range of ≥ 40 to ≤ 95 wt.-%, related to the total weight of the composition; wherein the at least one liquid hydrocarbon oil and at least one fatty acid oil are present in a weight ratio in the range of ≥ 1:1 to ≤ 140:1; wherein the composition comprises at least one solid fat and/or wax chosen from the group consisting of non-volatile hydrocarbons, solid hydrocarbon esters or mixtures thereof; and wherein the at least one solid fat and/or wax is present in an amount in the range of ≥ 5 to ≤ 60 wt.-%, related to the total weight of the composition.

In a preferred embodiment, the scalp microbiome is restored within five days, preferably within three days, after chemical hair treatment.

In a preferred embodiment, the chemical hair treatment is chosen from the group consisting of hair dyeing, hair bleaching, hair straightening and/or hair perming; preferably the chemical hair treatment is hair dyeing; more preferably the chemical hair treatment is oxidative hair dyeing.

In a preferred embodiment, the at least two liquid oils (being at least one liquid fatty acid oil and at least one liquid hydrocarbon oil) are present in a total amount in the range of ≥ 60 to ≤ 80 wt.-%, preferably in the range of ≥ 65 wt.-% to ≤ 75 wt.-%, related to the total weight of the composition.

In a preferred embodiment, the at least one solid fat and/or wax (chosen from the group consisting of non-volatile hydrocarbons, solid hydrocarbon esters or mixtures thereof) is present in an amount in the range of ≥ 20 to ≤ 40 wt.-%, preferably in the range of ≥ 25 wt.-% to ≤ 35 wt.-%, related to the total weight of the composition.

In a preferred embodiment, the at least one liquid fatty acid oil is chosen from the group consisting of pure fatty acids, pure fatty acid esters, fatty acid blends or their mixtures thereof. The liquid fatty acid oil may be chosen from the group consisting of caprylic acid, myristoleic acid, palmitoleic acid, sapienic acid, oleic acid, eicosenoic acid, linoleic acid, alpha-linoleic acid, gamma-linoleic acid, docosahexaenoic acid, stearidonic acid, ecosadienoic acid, docosadienoic acid, linolenic acid, eicosapentaenoic acid, caprylic/capric triglycerides, isostearyl isostearate, isononyl isononanoate, isopropyl myristate, isopropyl palmitate, cetearyl ethylhexanoate, almond oil, argan oil, apricot kernel oil, avocado oil, baobab oil, borage oil, borage seed oil, buckthorn oil, broccoli seed oil, buriti oil, blackberry seed oil, camellia seed oil, cranberry seed oil, candlenut oil, calendula oil, camelina seed oil, canola oil, castor oil, chia seed oil, carrot seed oil, coconut oil, corn oil, cotton seed oil, evening primrose oil, flaxseed oil, grapeseed oil, hemp oil, hazelnut oil, jojoba seed oil, kaya oil, linseed oil, macadamia nuts oil, macadamia seed oil, moringa oleifera seed oil, meadowfoam seed oil, marula oil, mink oil, mustard oil, moringa oil, neem oil, olive oil, oat seed oil, pumpkin seed oil, parsic oil, pomegranate seed oil, peanut oil, peppermint oil, peach kernel oil, papaya seed oil, pecan nut oil, perilla oil, pistachio oil, poppy seed oil, pumpkin seed oil, rapeseed oil, rosehip seed oil, rice bran oil, safflower oil, sasanqua oil, sacha inchi oil, sesame seed oil, sweet almond oil, soybean oil, sunflower oil, tea seed oil, tomato seed oil, tsubaki oil, turtle oil, walnut oil, wheat germ oil, yolk oil or mixtures thereof; preferably the liquid fatty acid oil is chosen from the group consisting of almond oil, argan oil, apricot kernel oil, avocado oil, baobab oil, borage oil, borage seed oil, buckthorn oil, broccoli seed oil, buriti oil, blackberry seed oil, camellia seed oil, cranberry seed oil, candlenut oil, calendula oil, camelina seed oil, canola oil, castor oil, chia seed oil, carrot seed oil, coconut oil, corn oil, cotton seed oil, evening primrose oil, flaxseed oil, grapeseed oil, hemp oil, hazelnut oil, jojoba seed oil, kaya oil, linseed oil, macadamia nuts oil, macadamia seed oil, moringa oleifera seed oil, meadowfoam seed oil, marula oil, mink oil, mustard oil, moringa oil, neem oil, olive oil, oat seed oil, pumpkin seed oil, parsic oil, pomegranate seed oil, peanut oil, peppermint oil, peach kernel oil, papaya seed oil, pecan nut oil, perilla oil, pistachio oil, poppy seed oil, pumpkin seed oil, rapeseed oil, rosehip seed oil, rice bran oil, safflower oil, sasanqua oil, sacha inchi oil, sesame seed oil, sweet almond oil, soybean oil, sunflower oil, tea seed oil, tomato seed oil, tsubaki oil, turtle oil, walnut oil, wheat germ oil, yolk oil or mixtures thereof; more preferably the liquid fatty acid oil is chosen from the group consisting of marula oil, argan oil, jojoba seed oil, sweet almond oil, baobab oil and macadamia nut oil; even more preferably the liquid fatty acid oil is marula oil.

In a preferred embodiment, the at least one liquid hydrocarbon oil is chosen from the group consisting of fluid paraffin, mineral oil, white paraffin oil, paraffin liquidum, isoparaffin, isododecane, isohexadecane, polybutene, squalane, tetradecane, dodecane, docosane, isoeicosane, hydrogenated polyisobutene, hydrogenated polydecene, hydrogenated didecene, C8-9 alkane, C9-12 alkane, C10-11 alkane, C11-12 alkane, C11-14 alkane/cycloalkane, C14-22 alkane, C12-17 alkane, C13-15 alkane, C15-19 alkane, C18-21 alkane, C21-28 alkane, squalene, octadecene, hexadecene, or mixtures thereof; preferably from the group consisting of mineral oil, isododecane or mixture thereof.

In a preferred embodiment, the least one liquid hydrocarbon oil and at least one liquid fatty acid oil are present in a weight ratio in the range of ≥ 1:1 to ≤ 140:1, preferably in the range of ≥ 30:1 to ≤ 110:1, more preferably in the range of ≥ 60:1 to ≤ 80:1.

In a preferred embodiment, at least one solid fat and/or wax is chosen from the group consisting of solid paraffin, vaseline, ozokerite, pristan, ceresin, petrolatum, microcrystalline wax, or mixtures thereof; preferably at least one solid fat and/or wax is petrolatum.

In a preferred embodiment, the cosmetic composition comprises at least one anti-inflammatory agent, at least one antioxidating agent, at least one thickener and/or rheology modifier, at least one fragrance or mixtures thereof.

In a preferred embodiment, the cosmetic composition is substantially non-aqueous.

In a preferred embodiment, the cosmetic composition has a viscosity at 25°C in the range of ≥ 10 to ≤ 140 mPa.s, preferably in the range of ≥ 20 to ≤ 120 mPa.s, more preferably in the range of ≥ 40 to ≤ 100 mPa.s.

In a preferred embodiment, the cosmetic composition is a leave-on composition.

In a preferred embodiment, the composition is applied before chemically treating hair (e.g., dyeing hair); preferably within 24h before chemically treating hair, more preferably within 6h before chemically treating hair, still more preferably within 1h before chemically treating hair, even more preferably within 15 minutes before chemically treating hair.

In a second aspect, the presently claimed invention relates to a method for chemically treating hair and skin comprising the following steps: applying the cosmetic composition as described herein; chemically treating hair; and rinsing of the chemically treated hair.

### Detailed description

Before the composition of the presently claimed invention and formulations of the presently claimed invention are described, it is to be understood that this invention is not limited to particular compositions and formulations described, since such compositions and formulation may, of course, vary. It is also to be understood that the terminology used herein is not intended to be limiting, since the scope of the presently claimed invention will be limited only by the appended claims.

If hereinafter a group is defined to comprise at least a certain number of embodiments, this is meant to also encompass a group which preferably consists of these embodiments only. Furthermore, the terms 'first', 'second', 'third' or 'a', 'b', 'c', etc. and the like in the description and in the claims, are used for distinguishing between similar elements and not necessarily for describing a sequential or chronological order. It is to be understood that the terms so used are interchangeable under appropriate circumstances and that the embodiments of the presently claimed invention described herein are capable of operation in other sequences than described or illustrated herein. In case the terms 'first', 'second', 'third' or '(A)', '(B)' and '(C)' or '(a)', '(b)', '(c)', '(d)', 'i', 'ii' etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

Furthermore, the ranges defined throughout the specification include the end values as well, *i.e.,* a range of 1 to 10 implies that both 1 and 10 are included in the range. For the avoidance of doubt, applicant shall be entitled to any equivalents according to applicable law.

In the following passages, different aspects of the presently claimed invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to 'one embodiment' or 'an embodiment' means that a particular feature, structure or characteristic described in connection with the embodiment is included in at least one embodiment of the presently claimed invention. Thus, appearances of the phrases 'in one embodiment' or 'in an embodiment' in various places throughout this specification are not necessarily all referring to the same embodiment, but may refer to the same embodiment. Further, as used in the following, the terms "preferably", "more preferably", "even more preferably", "most preferably" and "in particular" or similar terms are used in conjunction with optional features, without restricting alternative possibilities. Thus, features introduced by these terms are optional features and are not intended to restrict the scope of the claims in any way.

Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the presently claimed invention, and form different embodiments, as would be understood by those in the art. For example, in the appended claims, any of the claimed embodiments can be used in any combination.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

As used herein the term *"hair"* may be *"living", i.e.,* on a living body, or may be *"non-living", i.e.,* in a wig, hairpiece or other aggregation of non-living keratinous fibres. Mammalian, preferably human hair is preferred. However, wool, fur and other keratin fibres (keratin-containing fibres) are suitable substrates for the composition of the presently claimed invention.

*By "cosmetic composition"* is meant a composition for topical application to skin, hair and/or scalp of mammals, preferably human beings.

*By "haircare (scalp-care) composition"* is meant a composition for topical application to hair and/or scalp (scalp) of mammals, preferably human beings.

*By "microbiome"* is meant a community of microorganisms, including bacteria, yeasts, viruses, and fungi, that exist in given environment. By *"skin microbiome"* (or *"skin flora"*) is meant the community of microorganisms, including bacteria, yeasts, viruses, and fungi, that are present on skin. By *"scalp microbiome"* (or *"scalp flora"*) is meant the community of microorganisms, including bacteria, yeasts, viruses, and fungi, that are present on scalp.

*By "liquid oil"* is meant an oil, which is a lipophilic substance in liquid (non-solid or paste) form at room temperature (25°C) under atmospheric pressure (760 mmHg), with a logP value (natural logarithm of the partition coefficient of octanol-to-water) of greater than 0, more preferably greater than 3, and is preferably not an emulsifier or surfactant. A liquid oil may be volatile or non-volatile, preferably non-volatile. Presently, the terms "oil" and "lipophilic substance" are interchangeably used. Liquid oils encompass liquid fatty acid oils and liquid hydrocarbon oils.

By *"solid fat and*/*or wax"* is meant a fat and/or a wax, which is a lipophilic substance in solid form at room temperature (25°C) under atmospheric pressure (760 mmHg), with a logP value (natural logarithm of the partition coefficient of octanol-to-water) of greater than 0, more preferably greater than 3, and is preferably not an emulsifier or surfactant, and the melting range of the solid fat and/or wax may begin from below 25°C. Solid fats and/or waxes encompass non-volatile hydrocarbons and solid hydrocarbon esters.

*By "pure fatty acids"* is meant pure fatty acids (molecules), which may either be from synthetic origin or from natural origin after purification.

*By "fatty acid blend"* is meant a mixture of at least two fatty acids (and/or their derivatives such as fatty acid esters) from natural origin, particularly from plant origin.

*By "substantially free of'* is meant a composition comprising 0.1 wt.-% or less, preferably 0.01 wt.-% or less, more preferably about 0 wt.-%, of a compound, related to the total weight of the composition.

All percentages are by weight of the composition of the presently claimed invention, *i.e.,* of the ready-to-use composition, which is the composition to be applied on hair, unless otherwise specified. Also ratios are weight ratios unless specifically stated otherwise.

In an aspect, the presently claimed invention relates to the use of a cosmetic composition for maintaining the scalp microbiome during and/or after chemical hair treatment or restoring the scalp microbiome after chemical hair treatment; wherein the cosmetic composition comprises at least two liquid oils being at least one liquid fatty acid oil and at least one liquid hydrocarbon oil; wherein the at least two liquid oils are present in a total amount in the range of ≥ 40 to ≤ 95 wt.-%, related to the total weight of the composition; wherein the at least one liquid hydrocarbon oil and at least one fatty acid oil are present in a weight ratio in the range of ≥ 1:1 to ≤ 140:1; wherein the composition comprises at least one solid fat and/or wax chosen from the group consisting of non-volatile hydrocarbons, solid hydrocarbon esters or mixtures thereof; and wherein the at least one non-volatile hydrocarbon and/or solid hydrocarbon ester is present in an amount in the range of ≥ 5 to ≤ 60 wt.-%, related to the total weight of the composition. The cosmetic composition thus comprises at least one liquid fatty acid oil and at least one liquid hydrocarbon oil, which are present in a total amount in the range of ≥ 40 to ≤ 95 wt.-%, as well as at least one solid fat and/or wax, which is present in an amount in the range of ≥ 5 to ≤ 60 wt.-%, related to the total weight of the composition. It is also disclosed a method of maintaining the scalp microbiome during and/or after chemical hair treatment or restoring the scalp microbiome after chemical hair treatment, said method comprising the step of applying the cosmetic composition described herein onto hair and scalp. The inventors found that the at least two liquid oils (being at least one liquid fatty acid oil and at least one liquid hydrocarbon oil) and the at least one solid fat and/or wax (chosen from the group consisting of non-volatile hydrocarbons, solid hydrocarbon esters or mixtures thereof), when present in a cosmetic composition in the prescribed proportions, allow a quicker restoration of the scalp microbiome after chemical hair treatment.

*"Maintaining the scalp microbiome"* refers to the process of maintaining a balanced community of microorganisms, including bacteria, yeasts, viruses, and fungi, on the scalp. This maintenance aims to maintaining the scalp microbiome in the state where the microbiome is not significantly altered before and after an intervention. Measures to determine the maintenance of the scalp microbiome include assessing microbial diversity and abundance through indices such as the Shannon diversity index and the Simpson diversity index. Additionally, the absolute and relative abundance of specific beneficial microorganisms, like *Cutibacterium acnes* and *Staphylococcus capitis,* are monitored using techniques such as DNA extraction and quantitative PCR (qPCR). The effectiveness of maintenance can be quantified by comparing these microbial metrics before and after treatment, ensuring that the scalp microbiome is at a state of eubiosis, characterized by a healthy and diverse microbial community that is not perturbed by the intervention.

*"Restoring the scalp microbiome"* refers to the process of re-establishing a balanced community of microorganisms, including bacteria, yeasts, viruses, and fungi, on the scalp following a disturbance or imbalance caused by factors such as chemical hair treatments. This restoration aims to return the scalp microbiome to a state where non-pathogenic, beneficial microorganisms are prevalent, while pathogenic or harmful microorganisms are minimized. Measures to determine the restoration of the scalp microbiome include assessing microbial diversity and abundance through indices such as the Shannon diversity index and the Simpson diversity index. Additionally, the absolute and relative abundance of specific beneficial microorganisms, like *Cutibacterium acnes* and *Staphylococcus capitis,* are monitored using techniques such as DNA extraction and quantitative PCR (qPCR). The effectiveness of restoration can be quantified by comparing these microbial metrics before and after treatment, ensuring that the scalp microbiome returns to a state of eubiosis, characterized by a healthy and diverse microbial community. The expressions *"restoring the scalp microbiome", "balancing the scalp microbiome"* and *"restoring a balanced scalp microbiome"* may be used interchangeably.

It is also disclosed the use of the cosmetic composition for preventing or limiting the appearance of an imbalanced scalp microbiome (dysbiosis) during and/or after chemical hair treatment. It is also disclosed a method of preventing or limiting the appearance of an imbalanced scalp microbiome (dysbiosis) during and/or after chemical hair treatment, said method comprising the step of applying the cosmetic composition onto hair and scalp. The inventors found that the at least two liquid oils (being at least one liquid fatty acid oil and at least one liquid hydrocarbon oil) and the at least one solid fat and/or wax (being the at least one non-volatile hydrocarbon and/or solid hydrocarbon ester), when present in a cosmetic composition in the prescribed proportions, allow preventing or limiting the appearance of an imbalanced scalp microbiome (dysbiosis) during and/or after chemical hair treatment.

It is also disclosed the use of the cosmetic composition for maintaining or increasing the absolute and/or relative abundance of non-pathogenic microorganisms during and/or after chemical hair treatment; preferably for maintaining or increasing the absolute and/or relative abundance of *Cutibacterium acnes, Cutibacterium granulosum, Staphylococcus capitis,* and/or *Malassezia globosa* during and/or after chemical hair treatment. It is also disclosed a method of maintaining or increasing the absolute and/or relative abundance of non-pathogenic microorganisms during and/or after chemical hair treatment, said method comprising the step of applying the cosmetic composition onto hair and scalp. The inventors found that the at least two liquid oils (being at least one liquid fatty acid oil and at least one liquid hydrocarbon oil) and the at least one solid fat and/or wax (beong the at least one non-volatile hydrocarbon and/or solid hydrocarbon ester), when present in a cosmetic composition in the prescribed proportions, allow maintaining or increasing the absolute and/or relative abundance of non-pathogenic microorganisms during and/or after chemical hair treatment.

In a preferred embodiment, the scalp microbiome (a balanced scalp microbiome) is restored within five days, preferably within three days, after chemical hair treatment.

In a preferred embodiment, the chemical hair treatment is chosen from the group consisting of hair dyeing, hair bleaching, hair straightening and/or hair perming; preferably the chemical hair treatment is hair dyeing; still more preferably the chemical hair treatment is oxidative hair dyeing. Hair dyeing, hair bleaching, hair straightening and/or hair perming may be carried out using any conventional treatment methods and products.

Hair dyeing refers to the process of changing the color of hair using chemical or natural substances. This process involves the application of dyes or pigments to the hair shaft to achieve a desired color effect. Hair dyeing can be classified into three levels based on the permanence and depth of color change: Level 1 (temporary), Level 2 (semi-permanent), and Level 3 (oxidative).

Level 1 hair dyeing, known as temporary hair dyeing, involves applying colorants that coat the outer layer of the hair shaft without penetrating the cortex. These dyes are typically formulated to last until the next shampoo. The mechanism involves the dyes adhering to the hair cuticle, providing an immediate but non-lasting color change. The color effect lasts from a single wash to a few washes, depending on the formulation and hair porosity. Temporary hair dyes are often used for special occasions, experimental color changes, or short-term fashion statements. Products may include sprays, gels, and rinses.

Level 2 hair dyeing, referred to as semi-permanent hair dyeing, involves the use of dyes that partially penetrate the hair shaft, providing a longer-lasting color change compared to temporary dyes. These dyes typically do not contain ammonia or peroxide. The mechanism involves the dye molecules entering the cuticle and reaching the outer cortex layer, providing a more durable color that gradually fades over time. The color effect typically lasts for 4-12 washes, depending on hair condition, dye formulation, and frequency of washing. Semi-permanent hair dyes are suitable for individuals seeking a moderate commitment to color change, covering grey hair, or enhancing natural hair color. Commonly, these dyes are available in creams, foams, and liquids.

Level 3 hair dyeing, known as oxidative hair dyeing, involves the use of oxidative dyes that chemically alter the hair's natural pigment. This process requires a developer, typically hydrogen peroxide, to open the hair cuticle and allow the dye to penetrate deep into the cortex. The mechanism involves the dye molecules penetrating the cortex, where they undergo a chemical reaction to form larger, permanent color molecules that are trapped within the hair structure. The color effect is long-lasting and does not wash out, although it may fade slightly over time due to environmental factors and hair growth. Oxidative hair dyeing is ideal for individuals seeking a long-term color change, covering grey hair completely, or making significant color alterations. Permanent hair dyes are available in various formulations, including creams, gels, and liquids.

In a preferred embodiment, the at least two liquid oils being at least one liquid fatty acid oil and at least one liquid hydrocarbon oil are present in a total amount in the range of ≥ 60 to ≤ 80 wt.-%, preferably in the range of ≥ 65 wt.-% to ≤ 75 wt.-%, related to the total weight of the composition.

In a preferred embodiment, the at least one non-volatile hydrocarbon and solid hydrocarbon ester is present in an amount in the range of ≥ 20 to ≤ 40 wt.-%, preferably in the range of ≥ 25 wt.-% to ≤ 35 wt.-%, related to the total weight of the composition.

In a preferred embodiment, the at least two liquid oils (being at least one liquid fatty acid oil and at least one liquid hydrocarbon oil) and the at least one solid fat and/or wax (chosen from the group consisting of non-volatile hydrocarbons, solid hydrocarbon esters or mixtures thereof) may be present in a weight ratio in the range of ≥ 0.67:1 to ≤ 19:1, preferably in the range of ≥ 0.7:1 to ≤ 9:1, more preferably in the range of ≥ 1:1 to ≤ 6:1, even more preferably in the range of ≥ 2:1 to ≤ 4:1.

The at least one liquid fatty acid oil may be chosen from the group consisting of pure fatty acids *(i.e.,* 100 wt.-% related to the total weight of the fatty acid oil), pure fatty acid esters (*i.e.,* 100 wt.-% related to the total weight of the fatty acid oil), or fatty acid blends *(i.e.,* fatty acid oils from natural origin comprising at least two fatty acids and/or their derivatives such as fatty acid esters) or mixtures thereof.

Pure fatty acids may be chosen from the group consisting of caprylic acid, myristoleic acid, palmitoleic acid, sapienic acid, oleic acid, eicosenoic acid, linoleic acid, alpha-linoleic acid, gamma-linoleic acid, docosahexaenoic acid, stearidonic acid, ecosadienoic acid, docosadienoic acid, linolenic acid, eicosapentaenoic acid, or mixtures thereof.

Pure fatty acid esters may be chosen from the group consisting of caprylic/capric triglycerides, isostearyl isostearate, isononyl isononanoate, isopropyl myristate, isopropyl palmitate, cetearyl ethylhexanoate or mixtures thereof; preferably the fatty acid esters are caprylic/capric triglycerides.

The fatty acid blends may comprise at least two fatty acids and/or fatty acid esters; preferably at least two fatty acids and/or fatty acid esters present in an amount in the range of ≥ 50 to ≤ 100 wt.-%; more preferably at least two fatty acids and/or fatty acid esters present in an amount in the range of ≥ 60 to ≤ 100 wt.-%; even more preferably at least two fatty acids and/or fatty acid esters present in an amount in the range of ≥ 80 to ≤ 100 wt.-%, related to the total weight of the fatty acid oil. Fatty acid blends may be chosen from natural liquid fatty acid oils; preferably from the group consisting of almond oil, argan oil, apricot kernel oil, avocado oil, baobab oil, borage oil, borage seed oil, buckthorn oil, broccoli seed oil, buriti oil, blackberry seed oil, camellia seed oil, cranberry seed oil, candlenut oil, calendula oil, camelina seed oil, canola oil, castor oil, chia seed oil, carrot seed oil, coconut oil, corn oil, cotton seed oil, evening primrose oil, flaxseed oil, grapeseed oil, hemp oil, hazelnut oil, jojoba seed oil, kaya oil, linseed oil, macadamia nuts oil, macadamia seed oil, moringa oleifera seed oil, meadowfoam seed oil, marula oil, mink oil, mustard oil, moringa oil, neem oil, olive oil, oat seed oil, pumpkin seed oil, parsic oil, pomegranate seed oil, peanut oil, peppermint oil, peach kernel oil, papaya seed oil, pecan nut oil, perilla oil, pistachio oil, poppy seed oil, pumpkin seed oil, rapeseed oil, rosehip seed oil, rice bran oil, safflower oil, sasanqua oil, sacha inchi oil, sesame seed oil, sweet almond oil, soybean oil, sunflower oil, tea seed oil, tomato seed oil, tsubaki oil, turtle oil, walnut oil, wheat germ oil, yolk oil or mixtures thereof.

Marula oil comprises oleic acid, which is present in the range of ≥ 70 to ≤ 78 wt.-%. Additionally, linoleic acid is present in the range of ≥ 4 to ≤ 7 wt.-%, palmitic acid is present in the range of ≥ 9 to ≤ 12 wt.-%, stearic acid is present in the range of ≥ 5 to ≤ 8 wt.-%, and palmitoleic acid is present in the range of ≥ 0.7 to ≤ 1.5 wt.-%, based on the total weight of marula oil.

Argan oil comprises oleic acid, which is present in the range of ≥ 42 to ≤ 48 wt.-%. Additionally, linoleic acid is present in the range of ≥ 30 to ≤ 36 wt.-%, palmitic acid is present in the range of ≥ 10 to ≤ 15 wt.-%, and stearic acid is present in smaller amounts *i.e.,* in the range of ≤ 15 wt.-%, based on the total weight of marula oil.

Jojoba oil comprises eicosanoic acid, which is present in the range of ≥ 70 to ≤ 80 wt.-%. Additionally, oleic acid is present in the range of ≥ 10 to ≤ 15 wt.-%, and erucic acid is present in the range of ≥ 10 to ≤ 15 wt.-%, based on the total weight of jojoba oil.

Sweet almond oil comprises oleic acid, which is present in the range of ≥ 62 to ≤ 86 wt.-%. Additionally, linoleic acid is present in the range of ≥ 20 to ≤ 30 wt.-%, and palmitic acid is present in smaller amounts *i.e.,* in the range of ≤ 5 wt.-%, based on the total weight of sweet almond oil.

Macadamia nut oil comprises oleic acid, which is present in the range of ≥ 55 to ≤ 67 wt.-%. Additionally, palmitoleic acid is present in the range of ≥ 16 to ≤ 23 wt.-%, and linoleic acid is present in the range of ≥ 1 to ≤ 3 wt.-%, based on the total weight of macadamia nut oil.

Baobab oil comprises oleic acid, which is present in the range of ≥ 30 to ≤ 40 wt.-%. Additionally, linoleic acid is present in the range of ≥ 24 to ≤ 34 wt.-%, and palmitic acid is present in the range of ≥ 18 to ≤ 30 wt.-%, based on the total weight of baobab oil.

The liquid fatty acid oil (as comprising pure fatty acids, pure fatty acid esters and/or fatty acid blends) may therefore be chosen from the group consisting of caprylic acid, myristoleic acid, palmitoleic acid, sapienic acid, oleic acid, eicosenoic acid, linoleic acid, alpha-linoleic acid, gamma-linoleic acid, docosahexaenoic acid, stearidonic acid, ecosadienoic acid, docosadienoic acid, linolenic acid, eicosapentaenoic acid, caprylic/capric triglyceride, isostearyl isostearate, isononyl isononanoate, isopropyl myristate, isopropyl palmitate, cetearyl ethylhexanoate, almond oil, argan oil, apricot kernel oil, avocado oil, baobab oil, borage oil, borage seed oil, buckthorn oil, broccoli seed oil, buriti oil, blackberry seed oil, camellia seed oil, cranberry seed oil, candlenut oil, calendula oil, camelina seed oil, canola oil, castor oil, chia seed oil, carrot seed oil, coconut oil, corn oil, cotton seed oil, evening primrose oil, flaxseed oil, grapeseed oil, hemp oil, hazelnut oil, jojoba seed oil, kaya oil, linseed oil, macadamia nuts oil, macadamia seed oil, moringa oleifera seed oil, meadowfoam seed oil, marula oil, mink oil, mustard oil, moringa oil, neem oil, olive oil, oat seed oil, pumpkin seed oil, parsic oil, pomegranate seed oil, peanut oil, peppermint oil, peach kernel oil, papaya seed oil, pecan nut oil, perilla oil, pistachio oil, poppy seed oil, pumpkin seed oil, rapeseed oil, rosehip seed oil, rice bran oil, safflower oil, sasanqua oil, sacha inchi oil, sesame seed oil, sweet almond oil, soybean oil, sunflower oil, tea seed oil, tomato seed oil, tsubaki oil, turtle oil, walnut oil, wheat germ oil, yolk oil or mixtures thereof; preferably from the group consisting of almond oil, argan oil, apricot kernel oil, avocado oil, baobab oil, borage oil, borage seed oil, buckthorn oil, broccoli seed oil, buriti oil, blackberry seed oil, camellia seed oil, cranberry seed oil, candlenut oil, calendula oil, camelina seed oil, canola oil, castor oil, chia seed oil, carrot seed oil, coconut oil, corn oil, cotton seed oil, evening primrose oil, flaxseed oil, grapeseed oil, hemp oil, hazelnut oil, jojoba seed oil, kaya oil, linseed oil, macadamia nuts oil, macadamia seed oil, moringa oleifera seed oil, meadowfoam seed oil, marula oil, mink oil, mustard oil, moringa oil, neem oil, olive oil, oat seed oil, pumpkin seed oil, parsic oil, pomegranate seed oil, peanut oil, peppermint oil, peach kernel oil, papaya seed oil, pecan nut oil, perilla oil, pistachio oil, poppy seed oil, pumpkin seed oil, rapeseed oil, rosehip seed oil, rice bran oil, safflower oil, sasanqua oil, sacha inchi oil, sesame seed oil, sweet almond oil, soybean oil, sunflower oil, tea seed oil, tomato seed oil, tsubaki oil, turtle oil, walnut oil, wheat germ oil, yolk oil or mixtures thereof; more preferably from the group consisting of marula oil, argan oil, jojoba seed oil, sweet almond oil, baobab oil, macadamia nut oil or mixtures thereof; even more preferably the liquid fatty acid oil is marula oil.

A liquid hydrocarbon oil is defined as a fluid substance composed predominantly of hydrocarbons, which are organic compounds consisting entirely of hydrogen and carbon atoms. These oils are typically derived from petroleum or synthesized through various chemical processes. They remain in a liquid state at ambient temperatures and pressures and are characterized by their ability to flow freely. Their composition can vary widely, encompassing a range of molecular weights and structures, including alkanes, cycloalkanes, aromatic hydrocarbons, and their mixtures.

The liquid hydrocarbon oil may be chosen from the group consisting of fluid paraffin, mineral oil, white paraffin oil, paraffin liquidum, isoparaffin, isododecane, isohexadecane, polybutene, squalane, tetradecane, dodecane, docosane, isoeicosane, hydrogenated polyisobutene, hydrogenated polydecene, hydrogenated didecene, C8-9 alkane, C9-12 alkane, C10-11 alkane, C11-12 alkane, C11-14 alkane/cycloalkane, C14-22 alkane, C12-17 alkane, C13-15 alkane, C15-19 alkane, C18-21 alkane, C21-28 alkane, squalene, octadecene, hexadecene, or mixtures thereof; preferably from the group consisting of mineral oil, isododecane or mixture thereof.

Mineral oil is defined as a clear, odorless, and colorless oil derived from the distillation of petroleum. It is composed of a mixture of alkanes and cyclic paraffins and is known for its high purity and stability. Mineral oil is used extensively in cosmetic formulations due to its moisturizing and emollient properties, creating a protective barrier on the skin to prevent moisture loss.

In a preferred embodiment, the liquid fatty acid oil is marula oil, and the liquid hydrocarbon oil is mineral oil.

In a preferred embodiment, the at least one liquid hydrocarbon oil and at least one liquid fatty acid oil are present in the cosmetic composition in a weight ratio in the range of ≥ 1:1 to ≤ 140:1, preferably in the range of ≥ 30:1 to ≤ 110:1, more preferably in the range of ≥ 60:1 to ≤ 80:1.

In a preferred embodiment, the at least one solid fat and/or wax (chosen from the group consisting of non-volatile hydrocarbons, solid hydrocarbon esters or mixtures thereof) is chosen from the group consisting of solid paraffin, vaseline, ozocerite, pristan, ceresin, petrolatum, microcrystalline wax or mixtures thereof; preferably the non-volatile hydrocarbon and solid hydrocarbon ester is petrolatum.

In a preferred embodiment, the cosmetic composition is substantially free of any alternative solid fats and/or waxes *(i.e.,* being different from the group consisting of non-volatile hydrocarbons, solid hydrocarbon esters or mixtures thereof). The cosmetic composition may be substantially free of cacao fat, coconut oil, horse fat, hardened coconut fat, palm oil, tallow, sheep fat, hardened tallow, palm kernel oil, lard, ox bone fat, wood wax kernel oil, hardened castor oil; lanolin, kapok wax, lanolin acetate, isopropyl lanolin fatty acid, reduced lanolin, hard lanolin, POE lanolin alcohol ether, POE lanolin alcohol acetate, POE cholesterol ether, lanolin fatty acid polyethylene glycol, POE hydrogenated lanolin alcohol ether, phospholipids (such as lecithins, ceramides or mixtures thereof), beeswax, apple wax, candelilla wax, cotton wax, carnauba wax, bayberry wax, insect wax, whale wax, montan wax, rice bran wax, cane wax, jojoba wax, shellac wax or mixtures thereof.

Petrolatum is defined as a semi-solid mixture of hydrocarbons, primarily composed of saturated hydrocarbons, which are derived from petroleum. It is also known as petroleum jelly. Petrolatum exhibits properties of both solid fats and waxes, possessing a smooth, thick consistency that is solid at room temperature but melts upon application to the skin. Its composition includes a range of hydrocarbons with varying chain lengths, predominantly in the C16 to C32 range.

In a preferred embodiment, the composition further comprises at least one anti-inflammatory agent. The anti-inflammatory agent may be chosen from the group consisting of steroidal anti-inflammatory substances of the corticosteroid type (such as hydrocortisone, dexamethasone, dexamethasone phosphate, methyl prednisolone or cortisone), non-steroidal anti-inflammatory substances (such as oxicams, salicylates, acetic acid derivatives, fenamates, propionic derivatives, antranilic acid derivatives or avenathramides), naturally occurring anti-inflammatory mixtures or substances that alleviate itching and redness (such as extracts or fractions from camomile, *Aloe vera, Commiphora* species, *Rubia* species, willow, willow-herb, oats, *calendula,* arnica, St John's wort, honeysuckle, rosemary, *Passiflora incarnata,* witch hazel, ginger or *Echinacea*) or mixtures thereof; preferably chosen from the group consisting of extracts or fractions from camomile, *Aloe vera,* oats, *calendula,* arnica, honeysuckle, rosemary, witch hazel, ginger or *Echinacea,* and/or pure substances being alpha-bisabolol, apigenin, apigenin-7-glucoside, gingerols, shogaols, gingerdiols, dehydrogingerdiones, paradols, natural or naturally occurring avenanthramides (such as tranilast, avenanthramide A, avenanthramide B, avenanthramide C, non-natural), non-naturally occurring avenanthramides (such as dihydroavenanthramide D, dihydroavenanthramide E, avenanthramide D, avenanthramide E, avenanthramide F), boswellic acid, phytosterols, glycyrrhizin, glabridin and licochalcone A or mixtures thereof; most preferably the anti-inflammatory agent is bisabolol. The at least one anti-inflammatory agent may be present in an amount in the range of ≥ 0.01 to ≤ 1.0 wt.-%, related to the total weight of the composition.

In a preferred embodiment, the composition further comprises at least one antioxidating agent (antioxidant). The anti-oxidating agent may be chosen from the group consisting of water soluble anti-oxidants (such as vitamin C, glutathione, polyphenols including epigallocatechin gallate EGCG or their derivatives), fat soluble anti-oxidants (such as vitamin A, beta-carotene, ubiquinone, tocopherol, tocopheryl acetate) and their mixtures thereof; preferably from the group consisting of fat soluble anti-oxidants; more preferably the anti-oxidating agent is chosen from the group consisting of tocopherol, tocopheryl acetate or mixtures thereof. The at least one anti-oxidating agent may be present in an amount in the range of ≥ 0.01 to ≤ 1.0 wt.-%, related to the total weight of the composition.

In a preferred embodiment, the composition further comprises at least one thickener and/or rheology modifier. The thickener and/or rheology modifier may be a synthetic thickener and/or rheology modifier, a natural thickener and/or rheology modifier or mixtures thereof. The thickening and/or rheology agents may be chosen from the group consisting of xanthan gum, tara gum, guar, hydroxypropyl guar, scleroglucan, methylcellulose, ethyl cellulose, hydroxyethyl cellulose, carboxymethyl cellulose, hydroxypropylmethyl cellulose, microcrystalline cellulose, hydroxybutylmethyl cellulose, hydroxypropyl cellulose, hydroxyethyl ethyl cellulose, cetyl hydroxyethyl cellulose, N-vinylpyrollidone, Acrylates/Ceteth-20 Itaconate Copolymer, hydroxypropyl starch phosphate, polyethoxylated urethanes or polycarbamyl polyglycol ester such as PEG-150/Decyl/SMDI copolymer, PEG-150/Stearyl/SMDI copolymer, trihydroxystearin, acrylates copolymer or hydrophobically modified acrylate copolymers (such as Acrylates / Steareth-20 Methacrylate Copolymer), acrylates/steareth-20 methacrylate crosspolymer, acrylates/vinyl neodecanoate crosspolymer, acrylates/beheneth-25 methacrylate copolymer, acrylates/Clü-30 alkyl acrylate crosspolymer, and non-ionic amphophilic polymers comprising at least one fatty chain and at least one hydrophilic unit selected from polyether urethanes comprising at least one fatty chain. The at least one thickener and/or rheology modifier may be present in an amount in the range of ≥ 0.01 to ≤ 20 wt.-%, preferably in an amount in the range of ≥ 0.01 to ≤ 10 wt.-%, more preferably in an amount in the range of ≥ 0.01 to ≤ 5 wt.-%, related to the total weight of the composition.

In a preferred embodiment, the composition further comprises at least one fragrance. Any suitable fragrance may be used, for example acyclic sesquiterpenes, preferably farnesol.

In a preferred embodiment, the composition is substantially free of at least one of the following agents: fatty alcohols, surfactants (including non-ionic surfactants, anionic surfactants, cationic surfactants and/or amphoretic surfactants), cationic polymers, reducing agents, alkalizing agents (alkalizers), colouring agents (including direct dyes, pigments and/or oxidative dye precursors), and/or preservatives.

In a preferred embodiment, the composition is a substantially non-aqueous composition *i.e.,* is substantially free of water and/or any aqueous compounds.

In a preferred embodiment, the composition has a viscosity at 25°C in the range of ≥ 10 to ≤ 140 mPa.s, preferably in the range of ≥ 20 to ≤ 120 mPa.s, more preferably in the range of ≥ 40 to ≤ 100 mPa.s. The viscosity is determined as follows: using a suitable viscometer and spindle system e.g. Haake Rotational Viscotester VT550 with NV spindle, fill the sample cup with an appropriate amount of sample; conditioning the sample at 25°C for a suitable time *e.g.,* 10 minutes till equilibrium temperature is reached; setting the shear rate to 270.5 s⁻¹ and start the equipment, measuring the reading at 30 secs; and taking an average of at least 3 readings to determine the final viscosity.

In a preferred embodiment, the composition may be packaged and dispensed in any appropriate manner. For example, the composition may be packaged in a bottle. The composition may be dispensed as a spray using a pump, as an aerosol using a propellant, etc.

The cosmetic composition is a topical composition. In one embodiment, the composition is a rinse-off composition. By *"rinse-off'* is meant a composition, which is rinsed off after application onto hair, particularly before chemically treating hair. In an alternative embodiment, the composition is a leave-on composition. By *"leave-on"* is meant a composition, which is not rinsed off after application onto hair, particularly before chemically treating hair. The composition is preferably a leave-on composition.

Leave-on compositions for hair refer to formulations designed to be applied to the hair and/or scalp without the need for rinsing off after application. These compositions are intended to remain on the hair to provide various benefits such as conditioning, styling, protection, and treatment.

Leave-on compositions for hair can be chosen from the group consisting of conditioners, styling products, heat protectants, and treatment products. Each of these categories serves distinct purposes and provides various benefits to the hair and scalp.

Conditioners are leave-on products that are designed to provide moisture, detangle, and smooth the hair without the need to rinse them out. They help in maintaining hair health, improving manageability, and enhancing the overall appearance of the hair. Common forms of leave-on conditioners include leave-in sprays, creams, and serums that are applied to damp or dry hair.

Styling products include a variety of formulations such as gels, mousses, sprays, and creams that are used to shape, hold, and style the hair. These products are applied to either damp or dry hair and left on to achieve the desired styling effect. They provide hold, volume, texture, and definition, allowing for creative and controlled hairstyling.

Heat protectants are leave-on compositions designed to protect the hair from damage caused by heat styling tools like blow dryers, flat irons, and curling wands. These products form a protective barrier on the hair surface, preventing moisture loss, breakage, and other forms of thermal damage. Heat protectants often come in the form of sprays, creams, or serums that are applied before heat styling.

Treatment products are specialized leave-on formulations intended to address specific hair or scalp issues such as dandruff, hair thinning, or scalp irritation. These products may contain active ingredients like antifungals, anti-inflammatories, or growth stimulants to treat and improve hair and scalp conditions. Treatment products can be in the form of serums, oils, or medicated sprays that are applied directly to the scalp or hair.

In a preferred embodiment, the composition is applied onto hair before chemically treating hair, preferably within 24h before chemically treating hair, more preferably within 6h before chemically treating hair, still more preferably within 1h before chemically treating hair, even more preferably within 15 minutes before chemically treating hair.

In another aspect, the presently claimed invention relates to a method for chemically treating hair comprising the following steps: applying a cosmetic composition defined above on hair; chemically treating hair; and, rinsing the chemically treated hair.

The hair may be chemically treated using any conventional methods, particularly methods that may lead to a temporary, semi-permanent or a permanent alteration of hair. Such harsh treatments may therefore cause discomfort, irritation or even damages on hair and/or on skin. The step of chemically treating hair may be chosen from the group consisting of dyeing hair, bleaching hair, straightening hair and/or perming hair.

### Advantages

The presently claimed invention offers one or more of the following advantages:
- Protecting the skin, particularly the skin microbiome, against the detrimental effect of chemical hair treatment;
- Protecting the scalp, particularly the scalp microbiome, against the detrimental effect of hair dyeing;
- Preventing or at least limiting the scalp sensitization induced by chemical hair treatment, particularly hair dyeing;
- Preventing or at least limiting the appearance of an imbalanced scalp microbiome (dysbiosis) during and/or after chemically treating hair, particularly dyeing hair;
- Maintaining the scalp microbiome (a balanced scalp microbiome) during and/or after the chemical hair treatment, particularly the hair dyeing;
- Restoring the scalp microbiome (a balanced scalp microbiome) after the chemical hair treatment, particularly the hair dyeing;
- Providing a skincare (scalp-care) and haircare composition for treating scalp and hair before treatment, particularly before dyeing;
- Increasing or at least maintaining the absolute and/or relative abundance and the diversity in non-pathogenic microorganisms; and
- Increasing the recovery rate of the non-pathogenic microorganisms, preferably *Cutibacterium acnes, Cutibacterium granulosum, Staphylococcus capitis,* and/or *Malassezia globosa.*

### Embodiments

Embodiment 1 - Use of a cosmetic composition for restoring the scalp microbiome after hair treatment, wherein the cosmetic composition comprises at least one liquid oil present in an amount in the range of ≥ 40 to ≤ 95 wt.-% and at least one solid fat and/or wax present in an amount in the range of ≥ 5 to ≤ 60 wt.-%, related to the total weight of the composition.

Embodiment 2 - The use of the cosmetic composition, according to embodiment 1, wherein the scalp microbiome is restored within five days, preferably within three days, after hair treatment.

Embodiment 3 - The use of the cosmetic composition, according to any preceding embodiments, wherein the hair treatment is chosen from the group consisting of hair shampooing, hair conditioning, hair grooming, hair dyeing, hair bleaching, hair straightening and/or hair perming; preferably the hair treatment is hair dyeing; more preferably the hair treatment is oxidative hair dyeing.

Embodiment 4 - The use of the cosmetic composition, according to any preceding embodiments, wherein the at least one liquid oil is present in an amount in the range of ≥ 60 to ≤ 80 wt.-%, preferably in the range of ≥ 65 wt.-% to ≤ 75 wt.-%, related to the total weight of the composition.

Embodiment 5 - The use of the cosmetic composition, according to any preceding embodiments, wherein the at least one fat and/or wax is present in an amount in the range of ≥ 20 to ≤ 40 wt.-%, preferably in the range of ≥ 25 wt.-% to ≤ 35 wt.-%, related to the total weight of the composition.

Embodiment 6 - The use of the cosmetic composition, according to any preceding embodiments, wherein the at least one liquid oil is chosen from the group consisting of natural liquid oils (such as almond oil, argan oil, apricot kernel oil, avocado oil, baobab oil, borage oil, borage seed oil, buckthorn oil, broccoli seed oil, buriti oil, blackberry seed oil, camellia seed oil, cranberry seed oil, candlenut oil, calendula oil, camelina seed oil, canola oil, castor oil, chia seed oil, carrot seed oil, coconut oil, corn oil, cotton seed oil, evening primrose oil, flaxseed oil, grapeseed oil, hemp oil, hazelnut oil, jojoba seed oil, kaya oil, linseed oil, macadamia nuts oil, macadamia seed oil, moringa oleifera seed oil, meadowfoam seed oil, marula oil, mink oil, mustard oil, moringa oil, neem oil, olive oil, oat seed oil, pumpkin seed oil, parsic oil, pomegranate seed oil, peanut oil, peppermint oil, peach kernel oil, papaya seed oil, pecan nut oil, perilla oil, pistachio oil, poppy seed oil, pumpkin seed oil, rapeseed oil, rosehip seed oil, rice bran oil, safflower oil, sasanqua oil, sacha inchi oil, sesame seed oil, sweet almond oil, soybean oil, sunflower oil, tea seed oil, tomato seed oil, tsubaki oil, turtle oil, walnut oil, wheat germ oil, yolk oil, or mixtures thereof), mono-, di- and/or triglycerine liquid oils (such as trioctanoic acid glycerine, triisopalmitic acid glycerine, liquid lanolin, or mixtures thereof), ester liquid oils (such as cetyl octanoate, isopropyl myristate, hexyl laurate, or mixtures thereof), fatty acids (such as capric acid, caprylic acid, myristoleic acid, palmitoleic acid, sapienic acid, oleic acid, gondoic acid, eicosenoic acid, nervonic acid, paullinic acid, linoleic acid, alpha-linoleic acid, gamma-linoleic acid, calendic acid, docosahexaenoic acid, stearidonic acid, ecosadienoic acid, docosadienoic acid, linolenic acid, pinolenic acid, eicosapentaenoic acid, or mixtures thereof), liquid hydrocarbon oils (such as fluid paraffin, mineral oil, white paraffin oil, paraffin liquidum, isoparaffin, isododecane, isohexadecane, polybutene, squalane, tetradecane, dodecane, docosane, isoeicosane, hydrogenated polyisobutene, hydrogenated polydecene, hydrogenated didecene, C8-9 alkane, C9-12 alkane, C10-11 alkane, C11-12 alkane, C11-14 alkane/cycloalkane, C14-22 alkane, C12-17 alkane, C13-15 alkane, C15-19 alkane, C18-21 alkane, C21-28 alkane, squalene, octadecene, hexadecene, or mixtures thereof), or mixtures thereof; preferably from the group consisting natural liquid oils, fatty acids, liquid hydrocarbon oils, or mixtures thereof; more preferably from the group consisting of mineral oil and marula oil.

Embodiment 7 - The use of the cosmetic composition, according to any preceding embodiments, wherein the cometic composition comprises at least two liquid oils being at least one liquid fatty acid oil and at least one liquid hydrocarbon oil.

Embodiment 8 - The use according to embodiment 7, wherein the at least one liquid fatty acid oil is chosen from the group consisting of pure fatty acids, fatty acid blends or their mixtures thereof; preferably the liquid fatty acid oil is chosen from the group consisting of capric acid, caprylic acid, myristoleic acid, palmitoleic acid, sapienic acid, oleic acid, gondoic acid, eicosenoic acid, nervonic acid, paullinic acid, linoleic acid, alpha-linoleic acid, gamma-linoleic acid, calendic acid, docosahexaenoic acid, stearidonic acid, ecosadienoic acid, docosadienoic acid, linolenic acid, pinolenic acid, eicosapentaenoic acid, caprylic/capric triglycerides, isostearyl isostearate, isononyl isononanoate, isopropyl myristate, isopropyl palmitate, cetearyl ethylhexanoate, almond oil, argan oil, apricot kernel oil, avocado oil, baobab oil, borage oil, borage seed oil, buckthorn oil, broccoli seed oil, buriti oil, blackberry seed oil, camellia seed oil, cranberry seed oil, candlenut oil, calendula oil, camelina seed oil, canola oil, castor oil, chia seed oil, carrot seed oil, coconut oil, corn oil, cotton seed oil, evening primrose oil, flaxseed oil, grapeseed oil, hemp oil, hazelnut oil, jojoba seed oil, kaya oil, linseed oil, macadamia nuts oil, macadamia seed oil, moringa oleifera seed oil, meadowfoam seed oil, marula oil, mink oil, mustard oil, moringa oil, neem oil, olive oil, oat seed oil, pumpkin seed oil, parsic oil, pomegranate seed oil, peanut oil, peppermint oil, peach kernel oil, papaya seed oil, pecan nut oil, perilla oil, pistachio oil, poppy seed oil, pumpkin seed oil, rapeseed oil, rosehip seed oil, rice bran oil, safflower oil, sasanqua oil, sacha inchi oil, sesame seed oil, sweet almond oil, soybean oil, sunflower oil, tea seed oil, tomato seed oil, tsubaki oil, turtle oil, walnut oil, wheat germ oil, yolk oil or mixtures thereof.

Embodiment 9 - The use according to any embodiments 7 or 8, wherein the at least one liquid hydrocarbon oil is chosen from the group consisting of fluid paraffin, mineral oil, white paraffin oil, paraffin liquidum, isoparaffin, isododecane, isohexadecane, polybutene, squalane, tetradecane, dodecane, docosane, isoeicosane, hydrogenated polyisobutene, hydrogenated polydecene, hydrogenated didecene, C8-9 alkane, C9-12 alkane, C10-11 alkane, C11-12 alkane, C11-14 alkane/cycloalkane, C14-22 alkane, C12-17 alkane, C13-15 alkane, C15-19 alkane, C18-21 alkane, C21-28 alkane, squalene, octadecene, hexadecene, or mixtures thereof.

Embodiment 10 - The use according to any one of embodiments 7 to 9, wherein the least one liquid hydrocarbon oil and at least one liquid fatty acid oil are present in a weight ratio in the range of ≥ 1:1 to ≤ 140:1, preferably in the range of ≥ 30:1 to ≤ 110:1, more preferably in the range of ≥ 60:1 to ≤ 80:1.

Embodiment 11 - The use of the cosmetic composition, according to any preceding embodiments, wherein the at least natural and/or synthetic solid fat and/or wax is chosen from the group consisting of cacao fat, coconut oil, horse fat, hardened coconut fat, palm oil, tallow, sheep fat, hardened tallow, palm kernel oil, lard, ox bone fat, wood wax kernel oil, hardened castor oil; lanolin, kapok wax, lanolin acetate, isopropyl lanolin fatty acid, reduced lanolin, hard lanolin, POE lanolin alcohol ether, POE lanolin alcohol acetate, POE cholesterol ether, lanolin fatty acid polyethylene glycol, POE hydrogenated lanolin alcohol ether, non-volatile hydrocarbons and solid hydrocarbon esters (such as solid paraffin, vaseline, ozocerite, pristan, ceresin, petrolatum, microcrystalline wax), phospholipids (such as lecithins, ceramides or mixtures thereof), beeswax, apple wax, candelilla wax, cotton wax, carnauba wax, bayberry wax, insect wax, whale wax, montan wax, rice bran wax, kapok wax, cane wax, jojoba wax, shellac wax or mixtures thereof; preferably from the group consisting of non-volatile hydrocarbons and solid hydrocarbon esters; more preferably the solid fat and/or wax is petrolatum.

Embodiment 12 - The use of the cosmetic composition, according to any preceding embodiments, wherein the cosmetic composition comprises at least one anti-inflammatory agent, at least one anti-oxidizing agent, at least one thickener and/or rheology modifier or mixtures thereof.

Embodiment 13 - The use of the cosmetic composition, according to any preceding embodiments, wherein the cosmetic composition is substantially non-aqueous.

Embodiment 14 - The use of the cosmetic composition, according to any preceding embodiments, wherein the cosmetic composition has a viscosity at 25°C in the range of ≥ 10 to ≤ 140 mPa.s, preferably in the range of ≥ 20 to ≤ 120 mPa.s, more preferably in the range of ≥ 40 to ≤ 100 mPa.s.

Embodiment 15 - The use of the cosmetic composition, according to any preceding embodiments, wherein the cosmetic composition is a leave-on composition.

Embodiment 16 - The use of the cosmetic composition, according to any preceding embodiments, wherein the composition is applied before treating hair; preferably within 24h before hair dyeing, more preferably within 6h before treating hair, still more preferably within 1h before treating hair, even more preferably within 15 minutes before treating hair.

Embodiment 17 - A method for treating hair and skin comprising the following steps: applying the cosmetic composition according to any preceding embodiments; treating hair; and rinsing of the treated hair.

### Examples

The presently claimed invention is illustrated in detail by non-restrictive working examples which follow. More particularly, the test methods specified hereinafter are part of the general disclosure of the application and are not restricted to the specific working examples.

### Formulation

It is provided a composition (composition X), which is formulated by mixing the following compounds together (weight percentages related to the total weight of the composition):
- 69.15 % mineral oil;
- 29.70 % petrolatum;
- 1.00 % marula oil refined;
- 0.10 % bisabolol; and
- 0.05 % d-alpha tocopheryl acetate.

### Skin sensitization (protocol and results)

A stinging test was conducted to evaluate the effectiveness for the formulation to reduce stinging due to hair dyeing chemicals. 28 volunteers were recruited. The application area behind the ears was tape stripped twice. Treatment by either composition X or untreated control was randomly assigned to the left or right application area. The treatment was applied, followed by application of the hair dyeing chemicals. The stinging sensation was evaluated on a scale of 0 to 5, with 5 being the worst, at various time points for each application area. The mean of the maximum sensitization score recorded over the treatment period was evaluated for each treatment.

The results relating to the skin sensitization for Treatment X obtained are shown in table 1 below.

**Table 1**

| Treatment | Mean of maximum sensitization score |
|---|---|
| Untreated control | 1.34* |
| Treatment composition X | 0.379 |

| | |
|---|---|
| *(p<0.05) | |

The data shows that application of Composition X significantly reduces skin sensitization when applied before the hair dyeing chemicals. The difference was significant when compared to untreated.

### Microbiome analysis (protocol)

The performance of composition X was tested on 50 persons (split in two groups of 25 persons). Group A corresponded to the untreated control group, while group B was treated with Composition X (invention).

The following steps were implemented:
- The hair of each person of groups A and B was shampooed daily for seven days (D0-D6);
- Composition X was applied onto the hair of each person from group B on day 7 (D7), before hair dyeing (no composition X was applied on the hair of each person of group A);
- The hair of each person from groups A and B was dyed (same hair dyeing products/method) on day 7 (D7);
- The dyed hair of each person from groups A and B was rinsed by shampooing on day 7 (D7); and
- The hair of each person from groups A and B was shampooed daily for seven days (D8-D14).

The microbiome was analysed using a *in vivo* microbiome testing. The *in vivo* microbiome testing consisted in:
- Recruiting volunteers being healthy individuals aged 20 to 40;
- Assessing that the volunteers had no scalp or hair disorders such as dandruff or excessive hair loss, were not using any anti-dandruff or medicated shampoo, were non-smokers, were not using chronic medication, had no chemical hair treatments in the past 2 months, and had no known allergies to hair dye;

- Randomly assigning volunteers to each group;
- Requesting the volunteers to not wash their hair at least from 12 to 18 hours before the sampling procedure;
- Taking samples on the scalp by creating a hair parting using a sanitized comb to expose the scalp, then rubbing a sterile cotton swab moistened in sterile water up and down the parting 10 times, while twisting left and right (samples were taken from the left, right, and centre parting of the scalp);
- Cutting swab heads from a single person and timepoint and combined them into a single tube containing collection buffer; and
- Storing swabs at 4°C before DNA extraction.
5 microbiome samples were taken on each person's scalp on the following timepoints:
- Day 0 (D0);
- Day 7 before hair dyeing (D7-Pre);
- Day 7 after hair dyeing (D7-Post);
- Day 10 (D10); and,
- Day 14 (D14).

DNA was extracted using the qPCR method, which was performed using a commercial assay kit targeting specific scalp microbiota. Abundance data was recorded as genome copy/µL DNA volume. Quality control and DNA quality assessment were performed using Nanodrop.

Friedman tests were used to compare the diversity across different timepoints. Diversity indexes were calculated and ranked. The Friedman test statistic was calculated and compared to chi-squared distribution to determine significance. Post hoc tests were used to identify specific group differences when Friedman test indicated significance. Statistical testing was done on absolute abundances of individual microbiota using Dunn's multiple comparison test within the framework of Friedman test. Differences were considered statistically significant at p < 0.05 (marked with an asterix).

The Shannon diversity Index and the Simpson diversity Index are indicators of the microbial diversity, considering both the species richness and species evenness of the microbial community. Compared to the Shannon diversity index, the Simpson diversity index is more influenced by evenness than richness. Higher values indicate a higher diversity within the microbiome.

### Microbiome analysis (Shannon diversity index)

The results relating to the Shannon diversity index (bacterial species level) obtained are shown in table 2 below.

**Table 2**

| Groups | D0 | D7-Pre (p-value vs previous) | D7-Post (p-value vs previous) | D10 (p-value vs previous) | D14 (p-value vs previous) |
|---|---|---|---|---|---|
| A Untreated control | 0.6991 | 0.7334 (>0.9999) | 0.5683 (0.0316*) | 0.7264 (0.0128*) | 0.6998 (>0.9999) |
| B Treatment composition X | 0.7193 | 0.7415 (>0.9999) | 0.6369 (0.0949) | 0.7687 (0.0049*) | 0.7288 (>0.9999) |

The data shown in table 2 above can be analysed as follows:
- No significant difference at D7-Pre *versus* D0 for group A (see the p-value of >0.9999);
- No significant difference at D7-Pre *versus* D0 for group B (see the p-value of >0.9999);
- Significant difference at D7-Post *versus* D7-Pre for group A (see the p-value of 0.0316);
- No significant difference at D7-Post *versus* D7-Pre for group B (see the p-value of 0.0949);
- Significant difference of the Shannon diversity index at D10 *versus* D7-post (see the p-value of 0.0049)

Based on the results shown in table 2 and their analysis indicated above, the following conclusions can be drawn:
- Over the first 7 days (D0 to D7-Pre), prior hair dyeing, the microbial diversity was stable for both group A and group B;
- There was a downward trend in the Shannon diversity after hair dyeing in both groups A and B;
- The reduction in the Shannon diversity index after hair dyeing (as compared to before hair dyeing) is less pronounced in group B *versus* comparative group A; and
- The use of composition X helped to minimize the impact of hair dying on the Shannon diversity index in group B in a significant manner.

### Microbiome analysis (Simpson index)

The results relative to the Simpson index (bacterial species level) obtained are shown in table 3 below.

**Table 3**

| Groups | D0 | D7-Pre (p-value vs previous) | D7-Post (p-value vs previous) | D10 (p-value vs previous) | D14 (p-value vs previous) |
|---|---|---|---|---|---|
| A Untreated control | 0.3886 | 0.4136 (>0.9999) | 0.3354 (0.3967) | 0.4072 (0.1574) | 0.3966 (>0.9999) |
| B | 0.4184 | 0.4233 | 0.3741 | 0.4292 | 0.4108 |
| Treated composition X | | (>0.9999) | (0.3967) | (0.0068*) | (>0.9999) |

The data shown in table 3 above can be analysed as follows:
- No significant difference at D7-Pre *versus* D0 for group A (see the p-value of >0.9999);
- No significant difference at D7-Pre *versus* D0 for group B (see the p-value of >0.9999);
- No significant difference at D10 *versus* D7-Post for group A (see the p-value of 0.1574); and
- Significant difference at D10 *versus* D7-Post for group B (see the p-value of 0.0068).

Based on the results shown in table 3 and their analysis indicated above, the following conclusions can be drawn:
- Over the first 7 days (D0 to D7-Pre), prior hair dyeing, the microbial diversity was stable for both group A and group B;
- There was an upward trend in diversity after hair dyeing in both groups A and B;
- The use of composition X (group B) significantly increased the Simpson diversity within 3 days after hair dyeing;
- There was no significant increase when composition X was not used (group A); and
- The use of composition X helped to recover the bacterial diversity (as represented by the Simpson index) within 3 days after hair dyeing, following a decrease of the bacterial diversity due to hair dyeing.

### Microbiome analysis (mean absolute abundance of Cutibacterium species)

The results obtained relative to the absolute abundance of *Cutibacterium* species (genome copy/µL) are shown in table 4 below.

**Table 4**

| Groups | D0 | D7-Pre (p-value vs previous) | D7-Post (p-value vs previous) | D10 (p-value vs previous) | D14 (p-value vs previous) |
|---|---|---|---|---|---|
| A Untreated control | 6883.6832 | 5842.5056 (>0.9999) | 3733.4319 (0.2535) | 3805.4867 (>0.9999) | 6095.2675 (0.3967) |
| B Treated composition X | 10345.6581 | 4797.4129 (>0.9999) | 2400.0152 (0.1574) | 6313.9928 (0.0175*) | 6967.4044 (>0.9999) |

The data shown in table 4 above can be analysed as follows:
- No significant difference at D10 *versus* D7-Post for group A (see the p-value of >0.9999); and
- Significant difference at D10 *versus* D7-Post for group B (see the p-value of 0.0175).

Based on the results shown in table 4 and their analysis indicated above, the following conclusions can be drawn:
- There was an upward trend in the absolute abundance of *Cutibacterium* species after hair dyeing in both groups A and B;
- The use of composition X (group B) significantly increased the absolute abundance of *Cutibacterium* species within 3 days after hair dyeing (D10);
- The absolute abundance of *Cutibacterium* species was not significantly increased when no composition X was used (group A);
- The use of Composition X thus helped to recover the abundance of *Cutibacterium* species within 3 days after hair dyeing, which was decreased due to hair dyeing.

### Microbiome analysis (mean absolute abundance of Cutibacterium acnes)

The results obtained relative to the absolute abundance of *Cutibacterium acnes* (genome copy/µL) are shown in table 5 below.

**Table 5**

| Groups | D0 | D7-Pre (p-value vs previous) | D7-Post (p-value vs previous) | D10 (p-value vs previous) | D14 (p-value vs previous) |
|---|---|---|---|---|---|
| A Untreated control | 8215.4058 | 7017.7499 (>0.9999) | 4612.4486 (0.1574) | 4590.4275 (>0.9999) | 7116.5869 (0.2004) |
| B Treated composition X | 11206.1058 | 5471.9862 (>0.9999) | 2905.8666 (0.1574) | 7178.4020 (0.0128*) | 7832.9837 (>0.9999) |

The data shown in table 5 above can be analysed as follows:
- No significant difference at D10 *versus* D7-Post for group A (see the p-value of >0.9999); and
- Significant difference at D10 *versus* D7-Post for group B (see the p-value of 0.0126).

Based on the results shown in table 5 and their analysis indicated above, the following conclusions can be drawn:
- There was an upward trend in the absolute abundance of *Cutibacterium acnes* after hair dyeing in both groups A and B;
- The use of Composition X (group B) significantly increased the absolute abundance of *Cutibacterium acnes* within 3 days after hair dyeing (D10);
- The absolute abundance of *Cutibacterium acnes* was not significantly increased when no composition X was used (group A); and
- The use of composition X thus helped to recover the absolute abundance of *Cutibacterium acnes* within 3 days after hair dyeing, which was decreased due to hair dyeing.

### Microbiome analysis (mean absolute abundance of Cutibacterium granulosum)

The results obtained relative to the absolute abundance of *Cutibacterium granulosum* (genome copy/µL) are shown in table 6 below.

**Table 6**

| Groups | D0 | D7-Pre (p-value vs previous) | D7-Post (p-value vs previous) | D10 (p-value vs previous) | D14 (p-value vs previous) |
|---|---|---|---|---|---|
| A Untreated control | 326.4788 | 173.4123 (>0.9999) | 45.2814 (0.0014*) | 177.8838 (0.3182) | 183.2723 (>0.9999) |
| B Treated composition X | 201.3905 | 125.5089 (>0.9999) | 45.9900 (0.4910) | 355.3725 (0.0175*) | 220.0612 (>0.9999) |

The data shown in table 6 above can be analysed as follows:
- No significant difference at D10 *versus* D7-Post for group A (see the p-value of >0.9999); and
- Significant difference at D10 *versus* D7-Post for group B (see the p-value of 0.0175).

Based on the results shown in table 6 and their analysis indicated above, the following conclusions can be drawn:
- There was an upward trend in the absolute abundance of *Cutibacterium granulosum* after hair dyeing in both groups A and B;
- The use of composition X (group B) significantly increased the absolute abundance of *Cutibacterium granulosum* within 3 days after hair dyeing (D10);
- The absolute abundance of *Cutibacterium granulosum* was not significantly increased when no composition X was used (group A); and
- The use of composition X thus helped to recover the absolute abundance of *Cutibacterium granulosum* within 3 days after hair dyeing, which was decreased by hair dyeing.

### Microbiome analysis (mean absolute abundance of Staphylococcus capitis)

The results obtained relative to the absolute abundance of *Staphylococcus capitis* (genome copy/µL) are shown in table 7 below.

**Table 7**

| Groups | D0 | D7-Pre (p-value vs previous) | D7-Post (p-value vs previous) | D10 (p-value vs previous) | D14 (p-value vs previous) |
|---|---|---|---|---|---|
| A Untreated control | 524.8348 | 528.1783 (>0.9999) | 100.3652 (0.0007*) | 744.0743 (0.1574) | 597.9055 (>0.9999) |
| B Treated | 1827.8903 | 430.6196 (>0.9999) | 128.2570 (0.0556) | 1176.8977 (<0.0001*) | 913.8404 (>0.9999) |
| composition X | | | | | |

The data shown in table 7 above can be analysed as follows:
- No significant difference at D10 *versus* D7-Post for group A (see the p-value of 0.1574); and
- Significant difference at D10 *versus* D7-Post for group B (see the p-value of <0.0001).

Based on the results shown in table 7 and their analysis indicated above, the following conclusions can be drawn:
- There was an upward trend in absolute abundance of *Staphylococcus capitis* after hair dyeing in both groups A and B;
- The use of Composition X (group B) significantly increased the absolute abundance of *Staphylococcus capitis* within 3 days after hair dyeing (D10), which decreased due to hair dyeing;
- The absolute abundance of *Staphylococcus capitis* was not significantly increased when no composition X was used (group A);
- The use of composition X thus helped to recover the absolute abundance of *Staphylococcus capitis* within 3 days after hair dyeing, which was decreased by hair dyeing.

### Microbiome analysis (mean absolute abundance of Malassezia globosa)

The results obtained relative to the absolute abundance of *Malassezia globosa* (genome copy/µL) are shown in table 8 below.

**Table 8**

| Groups | D0 | D7-Pre (p-value vs previous) | D7-Post (p-value vs previous) | D10 (p-value vs previous) | D14 (p-value vs previous) |
|---|---|---|---|---|---|
| A Untreated control | 296.2072 | 488.0339 (0.6034) | 1.9404 (0.0001*) | 7.0735 (0.0729) | 38.8856 (>0.9999) |
| B Treated composition X | 41.6343 | 23.5252 (>0.9999) | 4.9394 (0.0001*) | 20.9675 (0.0316*) | 17.7023 (>0.9999) |

The data shown in table 8 above can be analysed as follows:
- No significant difference at D10 *versus* D7-Post for group A (see the p-value of 0.0729); and
- Significant difference at D10 *versus* D7-Post for group B (see the p-value of 0.0018).

Based on the results shown in table 8 and their analysis indicated above, the following conclusions can be drawn:
- There was an upward trend in the absolute abundance of *Malassezia globosa* after hair dyeing in both groups A and B;
- The use of composition X significantly increased the absolute abundance of *Malassezia globosa* within 3 days after hair dyeing (group B);.
- The absolute abundance of *Malassezia globosa* was not significantly increased when no composition X was used (group A); and
- The use of Composition X thus helped to recover the absolute abundance of Malassezia globosa within 3 days after hair dyeing, which was decreased due by hair dyeing.

### Microbiome analysis (results summary)

A summary of the results is shown in table 8 below. Table 8 summarizes the p-values of selected microbial abundances comparing between D10 and D7-Post for groups A and B.

**Table 8**

| | D10 vs D7-Post (p-value) | |
|---|---|---|
| Microorganisms | Group A Untreated Control | Group B Treated (with composition X) |
| *Cutibacterium sp.* | >0.9999 | 0.0175* |
| *Cutibacterium acnes* | >0.9999 | 0.0128* |
| *Cutibacterium granulosum* | 0.3182 | 0.0175* |
| *Staphylococcus capitis* | 0.1574 | 0.0001* |
| *Malassezia globosa* | 0.0729 | 0.0316* |

| | | |
|---|---|---|
| *Significance (p<0.05) | | |

Based on the results summary shown in table 8, the following conclusions can be drawn:
- When composition X was used prior to hair dyeing (group B), the abundances of various microbial species, which are natural inhabitants of the scalp, were significantly increased at D10 vs D7-Post;
- By comparison, when no composition X was used prior to hair dyeing (group A), there was no significant improvement in the microbial abundance; and
- The use of composition X prior to hair dyeing positively influences the recovery of the microorganisms that are affected by hair dyeing.

The data reported in the table demonstrates that the application of Composition X facilitates the recovery of a balanced microbiome (*versus* untreated control).

Variations and modifications of the invention and further embodiments thereof, in addition to those described herein, will become apparent to those skilled in the art from the full contents of this document. The subject matter herein contains information, exemplification and guidance that can be adapted to the practice of this invention in its various embodiments and equivalents thereof. It is intended that the appended claims cover all such variations, modifications, embodiments and equivalents.

## Claims

1. Use of a cosmetic composition for maintaining the scalp microbiome during and/or after chemical hair treatment or restoring the scalp microbiome after chemical hair treatment;
wherein the cosmetic composition comprises at least two liquid oils being at least one liquid fatty acid oil and at least one liquid hydrocarbon oil;
wherein the at least two liquid oils are present in a total amount in the range of ≥ 40 to ≤ 95 wt.-%, related to the total weight of the composition;
wherein the at least one liquid hydrocarbon oil and at least one fatty acid oil are present in a weight ratio in the range of ≥ 1:1 to ≤ 140:1;
wherein the composition comprises at least one solid fat and/or wax chosen from the group consisting of non-volatile hydrocarbons, solid hydrocarbon esters or mixtures thereof; and
wherein the at least one solid fat and/or wax is present in an amount in the range of ≥ 5 to ≤ 60 wt.-%, related to the total weight of the composition.

2. The use of the cosmetic composition, according to claim 1, wherein the scalp microbiome is restored within five days, preferably within three days, after chemical hair treatment.

3. The use of the cosmetic composition, according to any preceding claims, wherein the chemical hair treatment is chosen from the group consisting of hair dyeing, hair bleaching, hair straightening and/or hair perming; preferably the hair treatment is hair dyeing; more preferably the chemical hair treatment is oxidative hair dyeing.

4. The use of the cosmetic composition, according to any preceding claims, wherein the at least two liquid oils being at least one liquid fatty acid oil and at least one liquid hydrocarbon oil are present in a total amount in the range of ≥ 60 to ≤ 80 wt.-%, preferably in the range of ≥ 65 wt.-% to ≤ 75 wt.-%, related to the total weight of the composition.

5. The use of the cosmetic composition, according to any preceding claims, wherein the at least one solid fat and/or wax is present in an amount in the range of ≥ 20 to ≤ 40 wt.-%, preferably in the range of ≥ 25 wt.-% to ≤ 35 wt.-%, related to the total weight of the composition.

6. The use according to any preceding claims, wherein the at least one liquid fatty acid oil is chosen from the group consisting of pure fatty acids, pure fatty acid esters, fatty acid blends or their mixtures thereof; preferably the liquid fatty acid oil is chosen from the group consisting of caprylic acid, myristoleic acid, palmitoleic acid, sapienic acid, oleic acid, eicosenoic acid, linoleic acid, alpha-linoleic acid, gamma-linoleic acid, docosahexaenoic acid, stearidonic acid, ecosadienoic acid, docosadienoic acid, linolenic acid, eicosapentaenoic acid, caprylic/capric triglycerides, isostearyl isostearate, isononyl isononanoate, isopropyl myristate, isopropyl palmitate, cetearyl ethylhexanoate, almond oil, argan oil, apricot kernel oil, avocado oil, baobab oil, borage oil, borage seed oil, buckthorn oil, broccoli seed oil, buriti oil, blackberry seed oil, camellia seed oil, cranberry seed oil, candlenut oil, calendula oil, camelina seed oil, canola oil, castor oil, chia seed oil, carrot seed oil, coconut oil, corn oil, cotton seed oil, evening primrose oil, flaxseed oil, grapeseed oil, hemp oil, hazelnut oil, jojoba seed oil, kaya oil, linseed oil, macadamia nuts oil, macadamia seed oil, moringa oleifera seed oil, meadowfoam seed oil, marula oil, mink oil, mustard oil, moringa oil, neem oil, olive oil, oat seed oil, pumpkin seed oil, parsic oil, pomegranate seed oil, peanut oil, peppermint oil, peach kernel oil, papaya seed oil, pecan nut oil, perilla oil, pistachio oil, poppy seed oil, pumpkin seed oil, rapeseed oil, rosehip seed oil, rice bran oil, safflower oil, sasanqua oil, sacha inchi oil, sesame seed oil, sweet almond oil, soybean oil, sunflower oil, tea seed oil, tomato seed oil, tsubaki oil, turtle oil, walnut oil, wheat germ oil, yolk oil or mixtures thereof; more preferably the liquid fatty acid oil is chosen from the group consisting of almond oil, argan oil, apricot kernel oil, avocado oil, baobab oil, borage oil, borage seed oil, buckthorn oil, broccoli seed oil, buriti oil, blackberry seed oil, camellia seed oil, cranberry seed oil, candlenut oil, calendula oil, camelina seed oil, canola oil, castor oil, chia seed oil, carrot seed oil, coconut oil, corn oil, cotton seed oil, evening primrose oil, flaxseed oil, grapeseed oil, hemp oil, hazelnut oil, jojoba seed oil, kaya oil, linseed oil, macadamia nuts oil, macadamia seed oil, moringa oleifera seed oil, meadowfoam seed oil, marula oil, mink oil, mustard oil, moringa oil, neem oil, olive oil, oat seed oil, pumpkin seed oil, parsic oil, pomegranate seed oil, peanut oil, peppermint oil, peach kernel oil, papaya seed oil, pecan nut oil, perilla oil, pistachio oil, poppy seed oil, pumpkin seed oil, rapeseed oil, rosehip seed oil, rice bran oil, safflower oil, sasanqua oil, sacha inchi oil, sesame seed oil, sweet almond oil, soybean oil, sunflower oil, tea seed oil, tomato seed oil, tsubaki oil, turtle oil, walnut oil, wheat germ oil, yolk oil or mixtures thereof; even more preferably the liquid fatty acid oil is chosen from the group consisting of marula oil, argan oil, jojoba seed oil, sweet almond oil, baobab oil and macadamia nut oil; most preferably the liquid fatty acid oil is marula oil.

7. The use according to any preceding claims, wherein the at least one liquid hydrocarbon oil is chosen from the group consisting of fluid paraffin, mineral oil, white paraffin oil, paraffin liquidum, isoparaffin, isododecane, isohexadecane, polybutene, squalane, tetradecane, dodecane, docosane, isoeicosane, hydrogenated polyisobutene, hydrogenated polydecene, hydrogenated didecene, C8-9 alkane, C9-12 alkane, C10-11 alkane, C11-12 alkane, C11-14 alkane/cycloalkane, C14-22 alkane, C12-17 alkane, C13-15 alkane, C15-19 alkane, C18-21 alkane, C21-28 alkane, squalene, octadecene, hexadecene, or mixtures thereof; preferably from the group consisting of mineral oil, isododecane or mixture thereof.

8. The use according to any one of claims 7 to 9, wherein the least one liquid hydrocarbon oil and at least one liquid fatty acid oil are present in a weight ratio in the range of ≥ 1:1 to ≤ 140:1, preferably in the range of ≥ 30:1 to ≤ 110:1, more preferably in the range of ≥ 60:1 to ≤ 80:1.

9. The use of the cosmetic composition, according to any preceding claims, wherein the at least one solid fat and/or wax is chosen from the group consisting of solid paraffin, vaseline, ozocerite, pristan, ceresin, petrolatum, microcrystalline wax or mixtures thereof; preferably the non-volatile hydrocarbon and solid hydrocarbon ester is petrolatum.

10. The use of the cosmetic composition, according to any preceding claims, wherein the cosmetic composition comprises at least one anti-inflammatory agent, at least one anti-oxidizing agent, at least one thickener and/or rheology modifier, at least one fragrance or mixtures thereof.

11. The use of the cosmetic composition, according to any preceding claims, wherein the cosmetic composition is substantially non-aqueous.

12. The use of the cosmetic composition, according to any preceding claims, wherein the cosmetic composition has a viscosity at 25°C in the range of ≥ 10 to ≤ 140 mPa.s, preferably in the range of ≥ 20 to ≤ 120 mPa.s, more preferably in the range of ≥ 40 to ≤ 100 mPa.s.

13. The use of the cosmetic composition, according to any preceding claims, wherein the cosmetic composition is a leave-on composition.

14. The use of the cosmetic composition, according to any preceding claims, wherein the composition is applied before chemically treating hair; preferably within 24h before chemically treating hair, more preferably within 6h before chemically treating hair, still more preferably within 1h before chemically treating hair, even more preferably within 15 minutes before chemically treating hair.

15. A method for chemically treating hair and skin comprising the following steps:
- applying the cosmetic composition according to any preceding claims;
- chemically treating hair; and
- rinsing of the chemically treated hair.
